# EUROPEAN PATENT SPECIFICATION

(11) **EP 4 186 982 B1**
(45) Date of publication and mention of the grant of the patent: **18.02.2026**
(21) Application number: 21210099.4
(22) Date of filing: 24.11.2021
(51) Int. Cl.: C12Q 1/6869

(54) **SEQUENCING OF DNA BY SEQUENTIAL ADDITION/INCORPORATION OF 3' UNPROTECTED LABELED NUCLEOTIDES**
SEQUENZIERUNG VON DNA DURCH SEQUENZIELLE HINZUFÜGUNG/EINBINDUNG VON 3' UNGESCHÜTZTEN MARKIERTEN NUKLEOTIDEN
SÉQUENÇAGE D'ADN PAR ADDITION/INCORPORATION SÉQUENTIELLE DE 3' NUCLÉOTIDES ÉTIQUETÉS NON PROTÉGÉS

(43) Date of publication of application: 31.05.2023
(73) Proprietor: Miltenyi Biotec B.V. & Co. KG, 51429 Bergisch Gladbach (DE)
(72) Inventor: PINARD, Robert, 51429 Bergisch Gladbach (DE); PERBOST, Michel, 51429 Bergisch Gladbach (DE)
(74) Representative: Kisters, Michael Marcus

(56) References cited:
- US-A1- 2008 132 692
- US-A1- 2015 299 767
- US-A1- 2020 372 971
- US-A1- 2021 147 930
- US-B2- 10 584 377
- US-B2- 8 772 473

## Description

### BACKGROUND

Next generation sequencing (NGS) is an extremely powerful tool to study clinically relevant target sequences and gene-base panel tests are the first choice for studying DNA variants. Approaches such as sequencing by hybridization (Drmanac et al. (1998) Nat Biotechnol 16:54-58), sequence-specific detection of single-stranded DNA using engineered nanopores (Kasianowicz et al. (1996) Proc Natl Acad Sci USA 93:13770-13773), pyrosequencing (Ronaghi et al. (1998) Science 281:363-365), sequencing of single DNA molecules (Braslavsky et al. (2003) Proc Natl Acad Sci USA 100:3960-3964), sequencing by ligation (Shendure et al. (2005) Science 309:1728-1732) and rolonies/polonies (Mitra et al. (2003) Anal Biochem 320:55-65) per example, have been widely explored.

However, DNA sequencing by synthesis (SBS) is currently the method of choice for sequencing. In this approach, each sequencing cycle involves at least the steps 1) addition of protected nucleotides provided with cleavable fluorescent label; 2) addition of polymerase and incorporation of nucleotide to a oligonucleotide primed DNA or RNA; 3) detection of fluorescent label; 4) removal of fluorescent label; 5) de-protection of the incorporated nucleotide, wherein each step maybe followed by a washing step.

DNA sequencing-by-synthesis (SBS) chemistry with reversibly terminating nucleotides, uses polymerase as the main enzyme and has already been incorporated in several DNA sequencing systems with significant performance. There are still many challenges to improve the speed and the cost of the sequencing including the efficient recognition and incorporation by DNA polymerases of modified nucleotides containing chemically reversible linkers tethering the cleavable fluorophore to the base and capping the 3'-OH group of the deoxyribose-phosphate backbone. To this date no success has been reported for the incorporation of such a nucleotide with a regular DNA polymerase. The main reason is that the 3' position on the deoxyribose is located in the vicinity of the amino acid residues in the active site of the polymerase and therefore a modification in this area of the ribose ring impairs or reduces the incorporation of these modified nucleotides (Pelletier et al. (1994) Science 264:1891-1903).

To the contrary, a unique fluorescent dye linked to the 5-position of the pyrimidines (T and C) and the 7-position of purines (G and A) via a cleavable linker is more tolerable as they are not located in the close proximity of the residues of the active site. In addition, the capping group needs to be chemically removed between each cycle in order to allow the subsequent reversibly terminating nucleotides. Therefore the overall process is quite slow; for example a typical sequencing of a DNA/RNA with 100 nucleotide units takes about 9-15 hours.

In addition, modified DNA polymerases need to be used to tolerate highly extensive modifications, including small chemical moiety used to cap the 3'-OH group and necessary for the SBS approach, and for allowing these nucleotide analogues to be incorporated into the growing DNA strand.

US8772473 discloses a cyclic "sequencing by synthesis" method, wherein 'OH unprotected, labeled nucleotides are used for sequencing of DNA in a step-by step imaging cycle. The process disclosed here requires washing, imaging and de-lableing after each incorporation of a nucleotide.

Accordingly, object of the invention was to provide a much faster and simpler process for sequencing DNA or RNA libraries.

### SUMMARY

In the current invention, we are describing a method for sequencing DNA or RNA by an approach using sequential addition/incorporation of non 3'capped fluorescently labeled nucleotides.

Object of the invention is therefore a method for detecting at least a part of the sequence of RNA or DNA molecules comprising
a) providing sequentially the nucleotides A, T, C and G, each provided with at least one fluorescent dye via a cleavable linker in presence of a polymerase to the DNA or RNA molecule thereby incorporating the nucleotides to the DNA or RNA molecule; wherein the fluorescent dyes of the nucleotides have different emission maxima and wherein the nucleotides have an unprotected 3'OH position
b) removing unincorporated nucleotides after each incorporation step and before the next nucleotide is provided
c) detecting the incorporated nucleotides by excitation and detecting of the fluorescent emission radiation of the fluorescent dyes, thereby obtaining sequence information
d) removing the fluorescent dyes from the incorporated nucleotides by cleaving the cleavable linkers
**characterized in that** the RNA or DNA molecules are provided with a sequence of 4 to 50 A, T, C and G nucleotides, each provided with at least one fluorescent dye as calibration sequence for fluorescent emission radiation and wherein the fluorescent emission radiation of the incorporated nucleotides is detected with its relative intensity against the fluorescent emission radiation of the calibration sequence and in that the detection step c) is performed once the sequential steps a) and b) for the nucleotides A, T, C and G have been performed.

In this approach, individual nucleotides which are unprotected (also referred to as non-capped) at the 3'OH position of the deoxyribose-phosphate backbone are used sequentially in a set order and the extended DNA or RNA strand is imaged solely after all four nucleotides have been added by a polymerase.

The current invention takes advantage of the multiple incorporation cycle and the reduction of the number of images that needs to be taken.

### BRIEF DESCRIPTION OF THE DRAWINGS

Fig. 1 shows generic formulas of the nucleotides A, T, C and G, having an unprotected ("uncapped") 3'OH position used in the invention and a protected ("capped") 3'OH position
Fig. 2 shows one cycle of the method of the invention

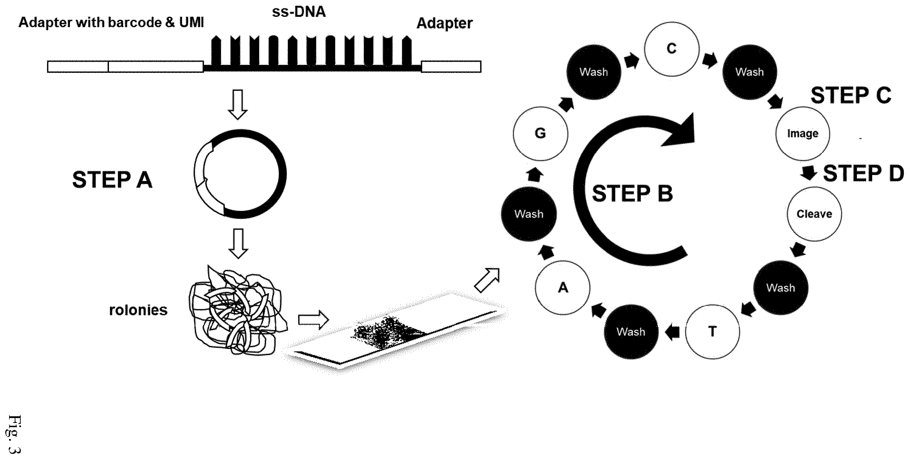

Fig. 3 shows an embodiment of the invention comprising providing the RNA/DNA molecule as rolony immobilized on a surface
Fig. 4 shows the calibration steps using mono incorporation during the first 4 cycles of sequencing using the last 4 nucleotides of the library adapter (calibration sequence) containing T,G,A and C.

### DETAILED DESCRIPTION

The usage of non-capped nucleotides allows for the usage of regular polymerases which in return eliminates the requirement for high temperature and genetically engineered thermophilic DNA polymerases currently used by SBS. These enzymes share the same catalytic mechanisms with their mesophilic counterparts but that optimally active at high temperatures.

Preferable, the nucleotides are incorporated to the DNA or RNA molecule in presence of a polymerase at a temperature between 10 - 80 °C, preferable in presence of a polymerase at a temperature between 10 - 40 °C

During SBS, the labels and terminators are chemically removed after incorporation and imaging in order to prepare the complementary strands for the next sequencing cycle. If during a cycle a base is not incorporated, a strand will start to lag behind. On the other hand, if multiple bases are added in a single cycle, the start will be ahead (lead). This is referred to as phasing. Phasing will make it more difficult to perform the actual base calling and would increase the error rate. The difficulty of incorporating the highly modified nucleotides especially with modifications at the 3' OH position of the deoxyribose phosphate backbone located in the vicinity of the active site of the polymerase would favor the phasing phenomena. The usage of no modification at the 3'OH position eliminates or vastly reduces the phasing issue.

Optionally, the RNA or DNA molecules are provided before the sequencing process with a sequence of 4 to 50 nucleotides as primer or adaptor for the polymerase.

For further identification purposes, it is possible to provide the RNA or DNA molecules before the sequencing process with a sequence of 4 to 50 nucleotides as unique identifier (UMI), optionally as part of the adapter sequence. Such sequences are known in the technical field of single cell sequencing.

In the invention, the RNA or DNA molecules are provided with a sequence of 4 to 50 A, T, C and G nucleotides, each provided with at least one fluorescent dye as calibration sequence for fluorescent emission radiation and wherein the fluorescent emission radiation of the incorporated nucleotides is detected with its relative intensity against the fluorescent emission radiation of the calibration sequence.

The calibration for each DNA/RNA molecule or rolony or cluster to be sequenced can be achieved by measuring the signal of the known calibration sequence. In the easiest variant, single incorporation of (one of each) A, T, C and G nucleotide during the first 4 cycles of the sequencing are used for calibration. To increase calibration efficiency, 2 - 4 of each A, T, C and G nucleotides may be provided as calibration sequence.

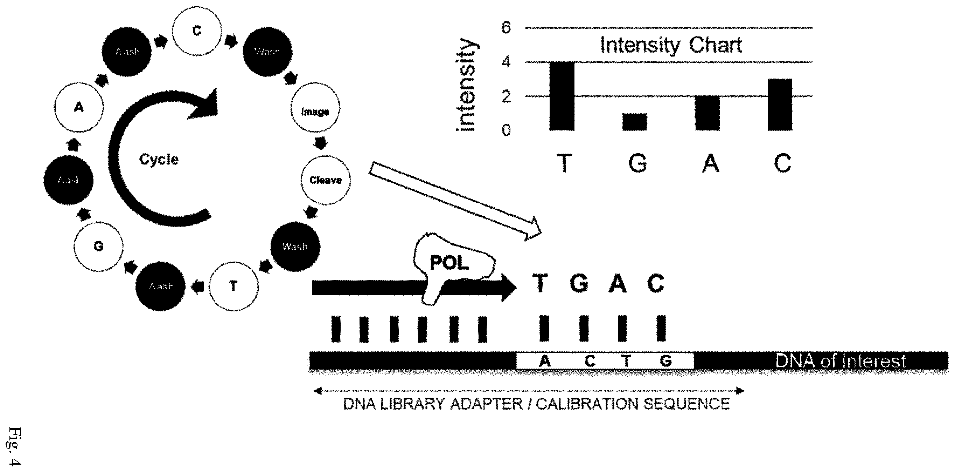

The general workflow of calibration process is shown in Fig. 4, wherein the measured intensities equal one nucleotide and wherein theses values are used to adjust the intensities of the respective nucleotides during sequencing process of the invention.

The signal of these mono incorporations performed for each rolony or cluster, before the actual sequencing the sequence of interest, allows the determination of the signal for a single nucleotide incorporation and to anticipate the expected signal for multiple base incorporation found in homopolymers.

In Step a) nucleotides A, T, C and G, each provided with at least one fluorescent dye via a cleavable linker are provided sequentially i.e. one sort of nucleotides after the other optionally followed by a washing procedure to remove not incorporated nucleotides.

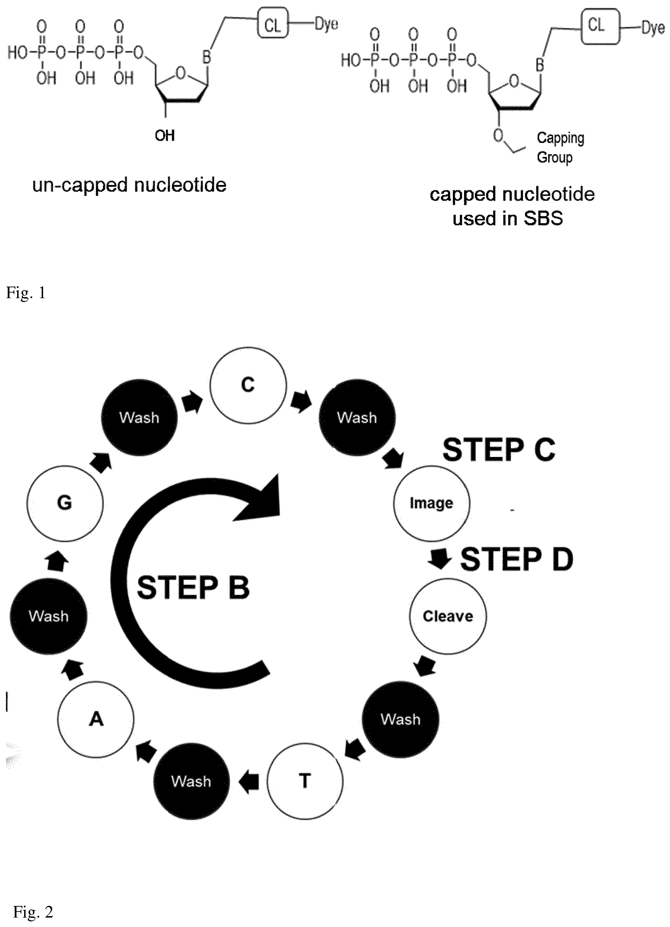

As shown in Fig. 1, the nucleotides used in the method of the invention have an unprotected 3'OH position.

The nucleotides may have the general formula (I) NP - (CL-D)x (I)
With N: natural or artificial nucleic acid
P: ≥3 phosphate groups
CL: cleavable linker
D: fluorescent dye
x: integer between 1 and 10

The nucleotides are provided in presence of a polymerase to the DNA or RNA molecule in a way that the nucleotides are incorporated to the DNA or RNA molecule.

In order to distinguish the incorporated nucleotides for detection, the fluorescent dyes of the nucleotides have different emission maxima.

It is further possible that each of the nucleotides A, T, C and G is provided as mixture comprising species provided with a fluorescent dyes via a cleavable linker and species without a fluorescent dye, wherein both species have an unprotected 3'OH position. With this embodiment, quenching of dyes adjacent to each other can be reduced.

Suitable fluorescent dyes and detection means are known the from the published sequencing technology. The nucleotides can be labeled using suitable fluorescent dyes known from the art of immunofluorescence technologies, e.g., flow cytometry or fluorescence microscopy. For example, fluorescent dyes are xanthene dyes, like fluorescein, or rhodamine dyes, coumarine dyes, cyanine dyes, pyrene dyes, oxazine dyes, pyridyl oxazole dyes, pyromethene dyes, acridine dyes, oxadiazole dyes, carbopyronine dyes, benzpyrylium dyes, fluorene dyes, or metallo-organic complexes, such as Ru, Eu, Pt complexes. Besides single molecule entities, clusters of small organic molecule dyes, fluorescent oligomers or fluorescent polymers, such as polyfluorene, can also be used as fluorescent moieties. Additionally, fluorescent dyes might be protein-based, such as phycobiliproteins, nanoparticles, such as quantum dots, upconverting nanoparticles, gold nanoparticles, dyed polymer nanoparticles. They can have modification on 3' or 5' end, or modification can be on any of the bases on the main backbone, such as dUTP with PA linker. The labeling molecules can be fluorescent molecules (R6G, ROX, Cy3, Cy5, Alexa dyes, ATTO dyes, etc) or can be energy transfer dyes.

The DNA or RNA molecules may be provided in the method in several variants. In a first variant, the DNA or RNA molecules may be provided as RNA or DNA rolonies comprising multiple concatemers of the RNA or DNA molecules. In a second variant, the RNA or DNA molecules are provided as single stranded RNA or DNA molecules and/or as a mixture of sense and anti-sense DNA single strands. In a third variant, the RNA or DNA molecules are fragmentated before the incorporation of nucleotides.

Further, the RNA or DNA molecules (optionally in form of rollonies) may be immobilized on a solid surface. RNA or DNA molecules can be immobilized by interacting with the surface via electrostatic charges or via NHS ester-activated crosslinkers.

RNA or DNA molecules/rollonies/nanoballs/clusters may be generated from libraries containing optionally calibration sequences, adapters, primers, UMIs or further barcodes. They possess several copies of a region of interest on a given genome and are attached to a solid surface or embedded in a gel.

In step a), non-capped fluorescent nucleotides (T, A, G or C) triphosphates are added to the solid surface in the presence of a DNA polymerase in a specific and known order. For example a solution containing T is added first followed by A, G and then C. Following each solution addition, the surface is washed to remove all trace of non-incorporated nucleotides.

In step c, after all incorporations, using the solutions containing all four nucleotides, the extended sequencing primers, corresponding to the region to be sequenced, are imaged in each respective nucleotide fluorescent channel.

In step d), the fluorescent dyes attached to the nucleotides base via a cleavable linker (for example a disulphide or azido bond containing linkers) are removed and the cycle is repeated starting again with step a).

The cleavable linkers may be enzymatically or chemically cleaved or cleaved by radiation.

The removal of the fluorescent dye on each nucleotides after the incorporation cycle depends on the cleavable linker and may be chosen as appropriate from the following:
- Disulfide linkers can be cleaved off selectively by treating with thiols leaving other linkers intact.
- Oxymethine-azide (-OCH(N₃)-) can be selectively cleaved off in the presence of photocleavable or other enzymatically cleavable linkers when treated with phosphines (e.g. TCEP).
- Alternatively, TCEP can be used to cleave off both disulfide and oxymethine azide linkers.
- Cleave reagents can be thiols (e.g. dithiothreitol - DDT, dimercapto-propane sulfonates - DMPS, etc), phosphines (e.g. TCEP, THPP, etc), mild reducing agents (e.g. Na2S2O4), specific wavelength of light, enzymes (e.g. peptidase, dextranase, esterase, phosphatase, proteinase etc)

Enzymatically cleavable linkers can be any molecule which can be cleaved by a specific enzyme like a hydrolase. Suitable as enzymatically degradable spacer P are, for example, polysaccharides, proteins, peptides, depsipeptides, polyesters, nucleic acids, and derivatives thereof. Suitable polysaccharides are, for example, dextrans, pullulans, inulins, amylose, cellulose, hemicelluloses, such as xylan or glucomannan, pectin, chitosan, or chitin, which may be derivatized to provide functional groups for covalent or non-covalent binding of the linker L Proteins, peptides, and depsipeptides used as enzymatically degradable linker can be functionalized via side chain functional groups of amino acids Polyesters and polyesteramides used as enzymatically degradable linker can either be synthesized with comonomers, which provide side chain functionality or be subsequently functionalized. In the case of branched polyesters functionalization can be via the carboxyl or hydroxyl end groups. Post polymerization functionalization of the polymer chain can be, for example, via addition to unsaturated bonds, i.e. thiolene reactions or azide-alkine reactions, or via introduction of functional groups by radical reactions

Steps b) to e) may be repeated until all the nucleotides required for the sequencing of at least a part or all of the DNA or RNA molecule to be sequenced is completed. For example, steps b) to e) are repeated in 5 to 1000 cycles.

## Claims

1. Method for detecting at least a part of the sequence of RNA or DNA molecules comprising
a) providing sequentially the nucleotides A, T, C and G, each provided with at least one fluorescent dye via a cleavable linker in presence of a polymerase to the DNA or RNA molecule thereby incorporating the nucleotides to the DNA or RNA molecule; wherein the fluorescent dyes of the nucleotides have different emission maxima and wherein the nucleotides have an unprotected 3'OH position
b) removing unincorporated nucleotides after each incorporation step and before the next nucleotide is provided
c) detecting the incorporated nucleotides by excitation and detecting of the fluorescent emission radiation of the fluorescent dyes, thereby obtaining sequence information
d) removing the fluorescent dyes from the incorporated nucleotides by cleaving the cleavable linkers
**characterized in that** the RNA or DNA molecules are provided with a sequence of 4 to 50 A, T, C and G nucleotides, each provided with at least one fluorescent dye as calibration sequence for fluorescent emission radiation and wherein the fluorescent emission radiation of the incorporated nucleotides is detected with its relative intensity against the fluorescent emission radiation of the calibration sequence and **in that** the detection step c) is performed once the sequential steps a) and b) for the nucleotides A, T, C and G have been performed.

2. Method according to claim 1 **characterized in that** steps b) to d) are repeated until all the nucleotides required for the sequencing of at least a part of the DNA or RNA molecule to be sequenced is completed

3. Method according to claim 1 **characterized in that** steps b) to d) are repeated in 5 to 1000 cycles.

4. Method according to any of the claims 1 to 3 **characterized in that** the cleavable linkers are enzymatically or chemically cleaved or cleaved by radiation.

5. Method according to any of the claims 1 to 4 **characterized in that** the RNA or DNA molecules are provided with a sequence of 4 to 50 nucleotides as unique identifier (UMI) as part of the adapter sequence.

6. Method according to any of the claims 1 to 5 **characterized in that** the nucleotides are incorporated to the DNA or RNA molecule in presence of a polymerase at a temperature between 10 - 80 °C

7. Method according to any of the claims 1 to 6 **characterized in that** the RNA or DNA molecules are provided as RNA or DNA rolonies comprising multiple concatemers of the RNA or DNA molecules.

8. Method according to any of the claims 1 to 6 **characterized in that** the RNA or DNA molecules are provided as single stranded RNA or DNA molecules and/or as a mixture of sense and anti-sense DNA single strands.

9. Method according to any of the claims 1 to 8 **characterized in that** the RNA or DNA molecules are fragmentated before the incorporation of nucleotides.

10. Method according to any of the claims 1 to 9 **characterized in that** the RNA or DNA molecules are immobilized on a solid surface

11. Method according to claim 10 **characterized in that** the RNA or DNA molecules are immobilized by interacting with the surface via electrostatic charges or via NHS ester-activated crosslinkers.

## Patentansprüche

1. Verfahren zum Nachweis mindestens eines Teils der Sequenz von RNA- oder DNA-Molekülen, umfassend
a) sequenzielles Bereitstellen der Nukleotide A, T, C und G, die jeweils mit mindestens einem Fluoreszenzfarbstoff über einen spaltbaren Linker bereitgestellt werden, in Gegenwart einer Polymerase zu dem DNA- oder RNA-Molekül, wodurch die Nukleotide in das DNA- oder RNA-Molekül eingebaut werden; wobei die Fluoreszenzfarbstoffe der Nukleotide unterschiedliche Emissionsmaxima aufweisen und wobei die Nukleotide eine ungeschützte 3'-OH-Position aufweisen,
b) Entfernen nicht eingebauter Nukleotide nach jedem Einbauschritt und vor der Bereitstellung des nächsten Nukleotids,
c) Nachweisen der eingebauten Nukleotide durch Anregung und Nachweis der Fluoreszenzemissionsstrahlung der Fluoreszenzfarbstoffe, wodurch Sequenzinformationen erhalten werden
d) Entfernen der Fluoreszenzfarbstoffe von den eingebauten Nukleotiden durch Spalten der spaltbaren Linker,
**dadurch gekennzeichnet, dass** die RNA- oder DNA-Moleküle mit einer Sequenz von 4 bis 50 Nukleotiden A, T, C und G bereitgestellt werden, die jeweils mit mindestens einem Fluoreszenzfarbstoff als Kalibrierungssequenz für Fluoreszenzemissionsstrahlung bereitgestellt werden, und wobei die Fluoreszenzemissionsstrahlung der eingebauten Nukleotide mit ihrer relativen Intensität gegenüber der Fluoreszenzemissionsstrahlung der Kalibrierungssequenz nachgewiesen wird, und dadurch, dass der Nachweisschritt (c) durchgeführt wird, sobald die sequenziellen Schritte a) und b) für die Nukleotide A, T, C und G durchgeführt worden sind.

2. Verfahren nach Anspruch 1, **dadurch gekennzeichnet, dass** die Schritte b) bis d) wiederholt werden, bis alle Nukleotide, die für die Sequenzierung mindestens eines Teils des zu sequenzierenden DNA- oder RNA-Moleküls erforderlich sind, vervollständigt sind.

3. Verfahren nach Anspruch 1, **dadurch gekennzeichnet, dass** die Schritte b) bis d) in 5 bis 1000 Zyklen wiederholt werden.

4. Verfahren nach einem der Ansprüche 1 bis 3, **dadurch gekennzeichnet, dass** die spaltbaren Linker enzymatisch oder chemisch oder durch Strahlung gespalten werden.

5. Verfahren nach einem der Ansprüche 1 bis 4, **dadurch gekennzeichnet, dass** die RNA- oder DNA-Moleküle mit einer Sequenz von 4 bis 50 Nukleotiden als eindeutige Kennung (UMI) als Teil der Adaptersequenz bereitgestellt werden.

6. Verfahren nach einem der Ansprüche 1 bis 5, **dadurch gekennzeichnet, dass** die Nukleotide in Gegenwart einer Polymerase bei einer Temperatur zwischen 10 und 80 °C in das DNA- oder RNA-Molekül eingebaut werden.

7. Verfahren nach einem der Ansprüche 1 bis 6, **dadurch gekennzeichnet, dass** die RNA- oder DNA-Moleküle als RNA- oder DNA-Rolonies bereitgestellt werden, die mehrere Konkatamere der RNA- oder DNA-Moleküle umfassen.

8. Verfahren nach einem der Ansprüche 1 bis 6, **dadurch gekennzeichnet, dass** die RNA- oder DNA-Moleküle als einzelsträngige RNA- oder DNA-Moleküle und/oder als Mischung aus Sense- und Antisense-DNA-Einzelsträngen bereitgestellt werden.

9. Verfahren nach einem der Ansprüche 1 bis 8, **dadurch gekennzeichnet, dass** die RNA- oder DNA-Moleküle vor dem Einbau von Nukleotiden fragmentiert werden.

10. Verfahren nach einem der Ansprüche 1 bis 9, **dadurch gekennzeichnet, dass** die RNA- oder DNA-Moleküle auf einer festen Oberfläche immobilisiert werden.

11. Verfahren nach Anspruch 10, **dadurch gekennzeichnet, dass** die RNA- oder DNA-Moleküle durch Wechselwirkung mit der Oberfläche über elektrostatische Ladungen oder über NHS-Esteraktivierte Vernetzer immobilisiert werden.

## Revendications

1. Procédé de détection d'au moins une partie de la séquence de molécules d'ARN ou d'ADN comprenant
a) la fourniture séquentiellement des nucléotides A, T, C et G, chacun fourni avec au moins un colorant fluorescent via un lieur clivable en présence d'une polymérase à la molécule d'ADN ou d'ARN incorporant ainsi les nucléotides dans la molécule d'ADN ou d'ARN, les colorants fluorescents des nucléotides ayant différents maxima d'émission et les nucléotides ayant une position 3'OH non protégée
b) l'enlèvement des nucléotides non incorporés après chaque étape d'incorporation et avant que le nucléotide suivant soit fourni
c) la détection des nucléotides incorporés par excitation et détection du rayonnement d'émission fluorescente des colorants fluorescents, obtenant ainsi des informations de séquence
d) l'enlèvement des colorants fluorescents des nucléotides non incorporés par clivage des lieurs clivables
**caractérisé en ce que** les molécules d'ARN ou d'ADN sont fournies avec une séquence de 4 à 50 nucléotides A, T, C et G, chacun fourni avec au moins un colorant fluorescent comme séquence de calibration pour rayonnement d'émission fluorescente et le rayonnement d'émission fluorescente des nucléotides incorporés étant détecté avec son intensité relative contre le rayonnement d'émission fluorescente de la séquence de calibration et **en ce que** l'étape de détection c) est effectuée une fois que les étapes séquentielles a) et b) pour les nucléotides A, T, C et G ont été effectuées.

2. Procédé selon la revendication 1, **caractérisé en ce que** les étapes b) à d) sont répétées jusqu'à ce que tous les nucléotides requis pour le séquençage d'au moins une partie de la molécule d'ARN ou d'ADN à séquencer soit complété.

3. Procédé selon la revendication 1, **caractérisé en ce que** les étapes b) à d) sont répétées dans 5 à 1 000 cycles.

4. Procédé selon l'une quelconque des revendications 1 à 3, **caractérisé en ce que** les lieurs clivables sont clivés enzymatiquement ou chimiquement ou clivés par rayonnement.

5. Procédé selon l'une quelconque des revendications 1 à 4, **caractérisé en ce que** les molécules d'ARN ou d'ADN sont fournies avec une séquence de 4 à 50 nucléotides comme identifiant unique (UMI) come partie de la séquence adaptatrice.

6. Procédé selon l'une quelconque des revendications 1 à 5, **caractérisé en ce que** les nucléotides sont incorporés dans la molécule d'ADN ou d'ARN en présence d'une polymérase à une température comprise entre 10 et 80 °C.

7. Procédé selon l'une quelconque des revendications 1 à 6, **caractérisé en ce que** les molécules d'ARN ou d'ADN sont fournies comme des colonies 'ARN ou d'ADN comprenant de multiples concatémères des molécules d'ARN ou d'ADN.

8. Procédé selon l'une quelconque des revendications 1 à 6, **caractérisé en ce que** les molécules d'ARN ou d'ADN sont fournies comme des molécules d'ARN ou d'ADN simple brin et/ou comme un mélange de simples brins d'ADN sens et anti-sens.

9. Procédé selon l'une quelconque des revendications 1 à 8, **caractérisé en ce que** les molécules d'ARN ou d'ADN sont fragmentées avant l'incorporation des nucléotides.

10. Procédé selon l'une quelconque des revendications 1 à 9, **caractérisé en ce que** les molécules d'ARN ou d'ADN sont immobilisées sur une surface solide.

11. Procédé selon la revendication 10, **caractérisé en ce que** les molécules d'ARN ou d'ADN sont immobilisées par interaction avec la surface via des charges électrostatiques ou via des réticulateurs activés par ester NHS.
